# NEW EUROPEAN PATENT SPECIFICATION

(11) **EP 0 633 784 B2**
(45) Date of publication and mention of the opposition decision: **17.10.2001**
(45) Mention of the grant of the patent: 27.12.1996
(21) Application number: 93906601.5
(22) Date of filing: 24.03.1993
(51) Int. Cl.: A61K 39/29, A61K 39/295

(54) **Hepatitis vaccins containing 3-0 deacylated monophosphoryl lipid A**
3-0-deacyliertes Monophosphoryl-Lipid-A enthaltende Hepatitis-Impfstoffe
Vaccins anti-hépatite contenant du monophosphoryl-lipide A 3-0-désacylé

(30) Priority: 27.03.1992 GB 9206786; 27.03.1992 GB 9206788; 27.03.1992 GB 9206789; 27.03.1992 GB 9206797
(43) Date of publication of application: 18.01.1995
(73) Proprietor: SMITHKLINE BEECHAM BIOLOGICALS S.A., 1330 Rixensart (BE)
(72) Inventor: GARCON-JOHNSON, Nathalie Marie-Josephe Claude, B-1330 Rixensart (BE); HAUSER, Pierre, SmithKline Beecham Biol. (s.a.), B-1330 Rixensart (BE); THIRIART, Clothilde, Smithline Beecham Biol.(s.a), B-1330 Rixensart (BE); VOET, Pierre, SmithKline Beecham Biol. (s.a.), B-1330 Rixensart (BE)
(74) Representative: Tyrrell, Arthur William Russell, Dr.
(86) International application number: EP9300712
(87) International publication number: WO9319780

(56) References cited:
- EP-A- 0 175 261
- EP-A- 0 318 216
- EP-A- 0 414 374
- EP-A- 0 419 182
- WO-A-89/12462
- WO-A-91/16926
- WO-A-92/11291
- GB-A- 2 220 211
- INFECTION AND IMMUNITY vol. 51, no. 3, 1986, WASHINGTON D.C.,USA pages 784 - 787 COURSAGET ET AL 'SIMULTANEOUS ADMINISTRATION OF DIPHTHERIA-TETANUS-PERTUSSIS-POLIO AND HEPATITIS B VACCINES IN A SIMPLIFIED IMMUNIZATION PROGRAM:IMMUNE RESPONSE TO DIPHTHERIA TOXOID,TETANUS TOXOID,PERTUSSIS, AND HEPATITIS B SURFACE ANTIGEN'
- K R Myers et al., "a critical determinant of lipid A endotoxic activity", in "Cellular and molecular aspects of endotoxin reactions"; A Novotny, JJ Spitzer, EJ Ziegler, Editors; 1990, pages 145-56.

## Description

The present invention relates to novel vaccine formulations, methods for preparing them and to their use in therapy. In particular the present invention relates to novel formulations for treating Hepatitis B infections and to combination vaccine formulations including a Hepatitis B vaccine component.

Viral hepatitis, caused by the A, B, C, D, and E hepatitis viruses, is a very common viral illness. Via the 3 and C viruses, in particular, it is also responsible for many cases of liver cancer. Thus the development of effective vaccines is critical and, despite notable successes. is still an on-going task. A review on modern hepatitis vaccines, including a number of key references, may be found in the Lancet. May 12th 1990 at page 1142 ff (Prof A.L.W.F. Eddleston). See also 'Viral Hepatitis and Liver Disease' (Vyas, B.N., Dienstag, J.L., and Hoofnagle, J.H., eds, Grune and Stratton, Inc. (1984) and 'Viral Hepatitis and Liver Disease' (Proceedings of the 1990) International Symposium, eds F.B. Hollinger, S.M. Lemon and H. Margolis, published by Williams and Wilkins).

Infection with hepatitis A virus (HAV) is a widespread problem but vaccines which can be used for mass immunisation are available, for example the product 'Havrix' (SmithKline Beecham Biologicals) which is a killed attenuated vaccine obtained from the HM-175 strain of HAV [see 'Inactivated Candidate Vaccines for Hepatitis A' by F.E. Andre, A Hepburn and E.D'Hondt, **Prog Med. Virol**. Vol 37, pages 72-95 (1990) and the product monograph 'Havrix' published by SmithKline Beecham Biologicals (1991)].

Flehmig et al (loc cit., pages 56-71) have reviewed the clinical aspects, virology, immunology and epidemiology of Hepatitis A and discussed approaches to the development of vaccines against this common viral infection.

In the present invention the hepatitis A antigen may comprise live attenuated virus particles or inactivated attenuated virus particles.

Infection with hepatitis B virus (HBV) is a widespread problem but vaccines which can be used for mass immunisation are now available, for example the product Engerix® -B' (SmithKline Beecham plc) which a obtained by genetic engineering techniques.

The preparation of Hepatitis B surface antigen (HBsAg) is well documented. See. for example, Harford et al in **Develop. Biol. Standard** 54, page 125(1983), Gregg et al in **Biotechnology**, 5, page 479(1987), EP-A-0 226 846, EP-A-0 299 108 and references therein.

As used herein the expression 'Hepatitis B surface antigen' or 'HBsAg' includes any HBsAg antigen or fragment thereof displaying the antigenicity of HBV surface antigen. It will be understood that in addition to the 226 amino acid sequence of the HBsAg S antigen (see Tiollais et al, Nature, 317, 489 (1985) and references therein) HBsAg as herein described may, if desired, contain all or part of a pre-S sequence as described in the above references and in EP-A-0 278 940. In particular the HBsAg may comprise a polypeptide comprising an amino acid sequence comprising residues 12-52 followed by residues 133-145 followed by residues 175-400 of the L-protein of HBsAg relative to the open reading frame on a Hepatitis B virus of ad serotype (this polypeptide is referred to as L∗; see EP 0 414 374). HBsAg within the scope of the invention may also include the preS1-preS2 -S polypeptide described in EP 0 198 474 (Endotronics) or analogues thereof such as those described in EP 0 304 578 (Mc Cormick and Jones). HBsAg as herein described can also refer to mutants, for example the 'escape mutant' described in WO 91/14703 or European Patent Application Publication Number 0 511 855 A1, especially HBsAg wherein the amino acid substitution at position 145 is to arginine from glycine.

Normally the HBsAg will be in particle form. The particles may comprise for example S protein alone or may be composite particles, for example (L*,S) where L* is as defined above and S denotes the S-protein of HBsAg. The said particle is advantageously in the form in which it is expressed in yeast.

Whilst experimental and commercially available Hepatitis vaccines, for example Havrix and Engerix® -B, afford excellent results it is an accepted fact that an optimal vaccine needs to stimulate not only neutralising antibody but also needs to stimulate as effectively as possible cellular immunity mediated through T-cells. There also exists a need for combination vaccines containing a Hepatitis component to stimulate cellular immunity in this way. The present invention achieves these aims.

WO 92/11291, which is part of the state of the art as defined in Article 54(3) EPC, discloses vaccine formulations that contain a hybrid polypeptide comprising a first polypeptide component which is HBsAg or fragment thereof covalently linked via a sulphur atom in the first polypeptide component to a second polypeptide component which is any antigen useful in the preparation of a polyvalent vaccine, in combination with 3-0-deacylated monophosphoryl lipid A.

The present invention provides a vaccine comprising a hepatitis B surface antigen wherein the hepatitis B surface antigen (HBsAg) is not part of an immunogenic hybrid polypeptide in conjunction with 3-O-deacylated monophosphoryl lipid A (abbreviated herein to 3-DMPL) and an aluminium salt.

3-O-deacylated monophosphoryl lipid A (or 3 De-O-acylated monophosphoryl lipid A) has formerly been termed 3D-MPL or d3-MPL to indicate that position 3 of the reducing end glucosamine is de-O-acylated For preparation, see GB 2 220 211 A Chemically it is a mixture of 3-deacylated monophcsphoryl lipid A with 4, 5 or 6 acylated chains. 'MPL' is a Registered Trademark of Ribi Immunochem., Montana which is used by Ribi to denote unambiguously their 3-O-deacylated monophosphoryl lipid A product. However, herein the terms 3D-MPL is used.

GB 2 220 211 A mentions that the endotoxicity of the previously used enterobacterial lipopolysacharides (LPS) is reduced while the immunogenic properties are conserved. However Go 2 220 211 cited these findings merely in con nection with bacterial (Gram negative) systems. At the priority date of the present invention the suitability of 3-Dea-cylated monophosphoryl lipid A as a adjuvant for a vaccine containing a hepatitis B surface antigen had not been suggested.

Surprisingly, however, it has been found that vaccine compositions according to the invention have particularly advantageous properties as described herein.

A particular advantage is that the vaccine formulations of the invention are very effective in inducing protective immunity, even with very low doses of antigen.

They provide excellent protection against primary infection and stimulate advantageously both specific humoral (neutralising antibodies) and also effector cell mediated (DTH) immune responses.

A further important advantage is that vaccine compositions according to the invention may also be used as therapeutic vaccines.

The 3D-MPL as defined above will normally be present in the range of 10-100µg, preferably 25-50µg per dose wherein the Hepatitis antigen will be typically present in a range 2-50µg per dose.

An embodiment of the invention is HBsAg S antigen (for example as in Engerix-B) in admixture with 3-DMPL and aluminium hydroxide as described hereinbelow.

Another embodiment of the invention additionally comprises HAV antigen (for example as in Havrix) in admixture with 3-DMPL and aluminium hydroxide as described hereinbelow.

A further specific embodiment of the invention is HBsAg antigen as (L*,S) particles, defined hereinabove, in admixture with 3-DMPL and aluminium hydroxide.

Other embodiments are given in the examples hereinbelow.

The invention in a further aspect provides a vaccine formulation as described herein for use in medical therapy, particularly for use in the treatment or prophylaxis of hepatitis B viral infections. In a preferred aspect the vaccine according to the invention is a therapeutic vaccine useful for the treatment of ongoing hepatitis infections, more especially hepatitis B infections in humans suffering therefrom.

In view of the surprisingly efficaceous results obtained, in a further preferred aspect the invention provides a vaccine composition for the treatment or prophylaxis of Hepatitis B infections.

Advantagously the hepatitis vaccine composition of the invention is admixed with other antigens so that it is effective in the treatment or prophylaxis of one or more other bacterial, viral or fungal infections.

Accordingly the hepatitis vaccine formulation according to the invention preferably is admixed with at least one other component selected from non-hepatitis antigens which are known in the art to afford protection against one or more of the following: diphtheria, tetanus, pertussis. Haemophilus influenaze b (Hib), and polio.

Particular combination vaccines (formed by admixture of antigens) within the scope of the invention include a DTP (diphtheria-tetanus-pertussis)-hepatitis B combination vaccine formulation, an Hib-Hepatitis B vaccine formulation, a DTP-Hib-Hepatitis B vaccine formulation and an IPV (inactivated polio vaccine) -DTP-Hib-Hepatitis B vaccine formulation.

The above combinations may advantageously include an admixed component which is protective against Hepatitis A, especially the killed attenuated stain derived from the HM-175 strain as is present in Havrix.

Suitable components for use in such vaccines are already commercially available and details may be obtained from the World Health Organisation. For example the IPV component may be the Salk inactivated polio vaccine. The pertussis vaccine may comprise whole cell or acellular product.

Advantageously the hepatitis or combination vaccine according to the invention is a paediatric vaccine.

Vaccine preparation is generally described in New Trends and Developments in Vaccines, edited by Voller et al., University Park Press. Baltimore, Maryland U.S.A. 1978. Encapsulation within liposomes is described, for example, by Fullerton, US Patent 4,235,877. Conjugation of proteins to macromoloecules is disclosed, for example, by Likhite, US Patent 4,372,945 and by Armor et al., US Patent 4,474,757.

The amount of antigen in each vaccine dose is selected as an amount which induces an immunoprotective response without significant, adverse side effects in typical vaccinees. Such amount will vary depending on which specific immunogens are employed. Generally it is expected that each dose will comprise 1-1000µg of total immunogen, preferably 2-100µg, most preferably 4-40µg. An optimal amount for a particular vaccine can be ascertained by standard studies involving observation of antibody titres and other responses in subjects. Following an initial vaccination, subjects may receive a boost in about 4 weeks.

In a further aspect of the present invention there is provided a method of manufacture of a vaccine effective in preventing or treating hepatitis infection, wherein the method comprises mixing the hepatitis B Surface antigen as defined herein with 3D-MPL and an aluminium salt.

Using this method one or more additional components are preferably admixed with HBsAg to provide a combination vaccine, advantageously for paediatric use.

The following examples illustrate the invention and its advantages.

### Example 1: Hepatitis B vaccine formulation

3D-MPL was obtained from Ribi Immunochem Research Inc. Aluminium hydroxide was obtained from Superfos (Alhydrogel).

3D-MPL was resuspended in water for injection at a concentration varying from 0.2 to 1 mg/ml by sonication in a water bath until the particles reach a size of between 80 and 500 nm as measured by photo correlation light scattering.

1 to 20µg of HBsAg (S- antigen as in Engerix® -B) in phosphate buffer solution at 1mg/ml) is adsorbed on 30 to 100µg of aluminium hydroxide (solution at 10.38 Al³⁺ mg/ml) for one hour at room temperature under agitation. To the solution is then added 30 to 50ug of 3D-MPL (solution 1 mg/ml). Volume and osmolarity are adjusted to 600µl with water for injection and phosphate buffer 5x concentrated. Solution is incubated at room temperature for 1 hour and kept at 4°C until use. Maturation of the formulation occurs during storage. This represents 10 injecting doses for testing in mice.

A similar formulation may be prepared by using as the HBsAg component the composite (L*,S) antigen as defined hereinabove.

### Example 2: Hepatitis A vaccine formulation

3D-MPL was obtained from Ribi Immunochem Research Inc. Aluminium hydroxide was obtained from Superfos (Alhydrogel).

HAV (360 to 22 EU per dose) is preadsorbed on 10% of the aluminium hydroxide final concentration (0.5mg/ml). 3D-MPL (12.5 to 100µg per dose) is added to the solution.

The remaining aluminium hydroxide is added to the solution and left for one hour at room temperature. Volumes are adjusted with phosphate buffer (phosphate 10mM, NaCl 150mM) and the final formulation is then stored at 4°C until use.

### Example 3: Combination vaccine formulation - Hepatitis B +Hepatitis A

HBsAg is adsorbed on 90% of the final amount of aluminium hydroxide (0.5mg/ml) and incubated overnight at room temperature. The pH is adjusted to 6.2 and the preparation is left 14 days at room temperature for maturation.

Hepatitis A antigen at 360 to 22EU per dose, in the form of an inactivated derivative of the HM-175 strain (as in Havrix) is preadsorbed on 10% of the aluminium hydroxide final concentration (0.5mg/ml). The remaining aluminium hydroxide is then added to the solution and left for one hour at room temperature under agitation.

The HAV adsorbed on aluminium hydroxide is then added to the HBsAg formulation.

3D-MPL is added to the HAV/HBsAg solution at a final concentration of 12.5 to 100µg per 1 ml dose, the volume is adjusted to the final dose volume, and the formulation is stored at 4°C until used.

### Example 4: Combination vaccines containing additional antigens

Combination vaccines containing DTP, IPV, Hib or acellular or whole cell pertussis antigens are prepared by adding one or more of the desired antigens to the formulations described in Example 1, Example 2 or Example 3 above.

### Example 5

### INCREASE OF HUMORAL IMMUNITY AND INDUCTION OF CELL MEDIATED IMMUNITY BY IMMUNIZATION OF MICE WITH HBsAg FORMULATED WITH ALUMINIUM HYDROXIDE AND 3D-MPL

### A. EFFECT OF Al(OH)3+ 3D-MPL ON INDUCTION OF ANTI-HBs ANTIBODIES

Balb/c mice were immunized by the subcutaneous route or by the intradermal route with recombinant HBsAg adsorbed on Al(OH)3 with 3D-MPL as adjuvant. Mice were immunized twice with HBsAg/Al/3D-MPL formulations and the antibody response was measured after the first and the second doses. Total lg were measured by ELISA or AUSAB kit (Abbott Lab, III.) and a particular attention was given to the induction of antibodies of the lgG2a isotype since this isotype is mainly induced by secretion of g-Interferon. The induction of this isotype thus indirectly reflects the activation of cell mediated immunity, namely the activation of Th1.

The ratio HBsAg/3D-MPL has been investigated as well as the size of 3D-MPL particles since suspension of 3D-MPL in water can result in particles of < 100 nm or > 500 nm.

### EXPERIMENT I - Effect of 3D-MPL (> 500 nm) dose on immunogenicity of rec.HBsAg adsorbed on Al(OH)3

Groups of 10 female Balb/c mice were injected by the subcutaneous route with 2.5 µg(mcg) of recHBsAg adsorbed on 50 µg(mcg) of Al+++ (as AI(OH)3) and increasing amounts of 3D-MPL (3.1 to 50µg(mcg)) with a particle size of > 500 nm. The mice were injected twice in a volume of 100 µl(mcl) and at 2 weeks interval. They were bled 2 weeks after the first injection (partial bleeding) and one week after the booster. Total anti-HBs IgG ad specific IgG2a were measured by ELISA using recHBsAg as capture antigen. The titers were expressed as the reciprocal of the dilution corresponding to 50 % of the maximal value (mid-point dilution). The results are given at table 1. They indicate an increase of both specific lgG and lgG2a with increasing dose of 3D-MPL, particularly for doses of 12.5 to 50 µg(mcg). The effect is seen for both primary and secondary responses and is particularly obvious for lgG2a (up to 20 fold increase) indirectly indicating a secretion of g-interferon induced by the immunization with 3D-MPL

### EXPERIMENT II - Comparison of clinical lots of adsorbed recHBsAg containing or not containing 3D-MPL (> 500 nm)

3 clinical lots of recHBsAg adsorbed on Al(OH)3 were prepared: lot DSAH16 contained no 3D-MPL and served as control. Lots DSAR501 and 502 were prepared in a similar way (20 µg(mcg) of recHBsAg adsorbed on 0.5 mg Al+++ as Al(OH)3) but contained 50 µg(mcg) of 3D-MPL (> 500 nm).

The 3 lots were injected subcutaneously to groups of 10 mice (200 µl(mcl) containing 2.5 µg(mcg) HBsAg, 100 µg(mcg) Al+++ and 6.25 µg(mcg) 3D-MPL), at 2 weeks interval. The mice were bled at day 14 and 1 week after the booster. Anti-HBs antibodies were measured using AUSAB kit or an in-house ELISA for either IgG or IgG2a The results are given in table 2. They indicate that, 2 weeks after the first injection, the 2 lots containing 3D-MPL induce a very significant anti-HBs response (12.4 and 41.9 mlU/ml) while the lot which does not contain 3D-MPL only induced a marginal response (0.75 mlU/ml). The number of responders is also higher with 3D-MPL (9/10 and 9/10 versus 1/10 in absence of 3D-MPL). The effect of 3D-MPL is confirmed after the booster since the titers obtained for lots DSAR501 and 502 are about 6 fold higher than that observed without 3D-MPL.

This indicates that, at least in mice. 3D-MPL (> 500 nm) can improve both the kinetics of the anti-HBs response and the level of the anti-HBs response.

These results were confirmed when specific IgG and IgG2a are measured after immunization with lots DSAH16 (without 3D-MPL) and DSAR502 (with 3D-MPL): the anti-HBs IgG titer is 5 (primary response) and 3 (secondary response) times higher when 3D-MPL is present.

For the IgG2a response, the effect of 3D-MPL is even more striking, at least after the second dose, indicating a preferential induction of IgG2a. This indirectly reflects activation of cell-mediated immunity (secretion of g-interferon) by the preparation containing 3D-MPL

### EXPERIMENT III - Effect of 3D-MPL (< 100 nm) dose on immunogenicity of recHBsAg adsorbed on Al(OH)3

Because 3D-MPL is much easier to obtain in a reproducible way as < 100 nm than as > 500 nm particles, the effect of < 100 nm 3D-MPL particles on the immunogenicity of recHBsAg previously adsorbed on Al(OH)3 was investigated.

Groups of 10 mice (Balb/c, female, 7 weeks old) were injected subcutaneously with 1 µg(mcg) of recHBsAg adsorbed on 50 µg(mcg) of Al+++ (as Al(OH)3) and in presence of increasing amounts (3.1 to 25 µg(mcg) of 3D-MPL (< 100 nm). The mice were injected twice at 2 weeks interval with a volume of 200 µl(mcl). They were bled 2 weeks after the first injection and 1 week after the booster. The anti-HBs response was evaluated by ELISA (total Ig, IgG. IgG2a) on pooled sera. The titers were expressed as mid-point dilutions (reciprocal of the dilution giving 50 % of the highest values). The results indicate that as few as 3.1 µg(mcg) of 3D-MPL induce a strong increase of the antibody response both for primary and secondary responses. The response culminates for 6.25 µg(mcg) and decreases afterwards to become similar to that found without 3D-MPL when high doses 3D-MPL (25 µg(mcg)) are used. The pattern of the antibody response is similar for IgG, IgG2a and total lg. It contrasts with results obtained for 3D-MPL of higher size (> 500 nm) (see experiment I) and suggests that small size (< 100 nm) particles of 3D-MPL are more effective than larger size (> 500 nm) particles (at least for humoral immunity), since less 3D-MPL is needed to obtain the maximal effect. The highest activity of small size 3D-MPL was confirmed in several experiments.

As shown for larger size 3D-MPL (> 500 nm), the adjuvant effect of 3D-MPL is higher for IgG2a than for total IgG or lg. At the maximal effect of the secondary response (6.25 µg(mcg) of 3D-MPL), there is a 25 fold increase for lgG2a while the increase for IgG or total lg was 7.6 and 4.3 respectively.

### B. INDUCTION OF CELL-MEDIATED IMMUNITY BY recHBsAg ADSORBED ON Al(OH)3 - EFFECT OF 3D-MPL

If humoral immunity is sufficient to protect against Hepatitis B, the induction of cell-mediated immunity (CTL. Th1) could be of particular importance for the treatment of the disease.

New formulations are required however for therapeutic vaccines since Al(OH)3 is capable of improving humoral immunity but not cell mediated immunity.

We have investigated the effect of 3D-MPL on the induction of Th1 cells capable of secreting IL-2 and g-(i.e. gamma) interferon in Balb/c mice immunized with recHBsAg adsorbed on Al(OH)3.

### EXPERIMENT I - Effect of 3D-MPL (> 500 nm) on induction of Th1 cells after immunization of Balb/c mice with Al(OH)3 adsorbed HBsAg

A group of 10 Balb/c mice (female, 5 weeks old) were immunized by injection in each footpad of 30 µl(mcl) containing 10 µg(mcg) of HBsAg, 15 µg(mcg) of Al+++ (as Al(OH)3) and 15 µg(mcg) of 3D-MPL. Control mice were injected similarly with the same amount of recHBsAg either mixed with FCA (positive control) or adsorbed on Al(OH)3 without 3D-MPL (negative control).

Six days after the immunization, the mice were killed and the popliteal lymph nodes were removed. The lymph node cells (LNC 2.105/ml) were cultivated for different periods of time (24 h to 74 h) in RPMI medium supplemented with 1 % of negative mouse serum and containing 5 µg(mcg)/ml of recHBsAg. After termination of the culture. the amount of IL-2, INF-g and IL-4 secreted in the medium was measured. IL-2 was estimated by its ability to stimulate the proliferation (evaluated by incorporation of 3H-Thymidine) of an IL-2-dependent CTL line (VDA2 cells) and the titer was express as stimulation index (SI = amount of 3H-Thymidine incorporated in stimulated cells/amount of 3H-Thymidine incorporated in non stimulated cells). The amount of IL-4 and INF-g was measured using commercial ELISA kits (Holland Biotechnology for IFN-g and Endogen for IL-4). The titers were expressed in pg of IFN-g/ml.

The results indicate that no significant amount of IL-2, IL-4 or INF-g is secreted by LNC from mice immunized with HBsAg adsorbed on Al(OH)3. On the contrary, high levels of IL-2 (I.S. = 38 at 48 h) and a significant amount of INF-g are secreted by LNC from mice immunized with HBsAg adsorbed on Al(OH)3+ 3D-MPL This secretion is similar (INF-g) or higher (IL-2) to that observed for mice immunized with HBsAg + FCA and the in vitro secretion occurs earlier.

No IL-4 was detected after immunization with HBsAg adsorbed on Al(OH)3 even in presence of 3D-MPL.

This secretion profile indicates that specific Th1 cells (IL-2, INF-g) have been induced by immunization with adsorbed HBsAg in presence of 3D-MPL but not in absence of 3D-MPL. However, no Th2 (IL-4) were detected in these conditions of immunization.

### EXPERIMENT II - Effect of the dose of 3D-MPL (< 100 nm) on the induction of Th1 cells after immunization of Balb/c mice with Al(OH)3 adsorbed recHBsAg

Groups of 5 Balb/c mice were immunized in each of the 2 footpads with 30 µl(mcl) containing 10 µg(mcg) of recHBsAg adsorbed on 15 µg(mcg) of Al+++ (as Al(OH)3) and with increasing amounts of 3D-MPL (100 nm, 0 to 15 µg(mcg)).

Six days after the injection, the mice were killed and the popliteal lymph node cells (LNC) were cultivated at 2.106 cells/ml in RPMI supplemented with 1 % negative mouse serum for different periods of time (24 h to 96/25) in presence of 5 µg(mcg)/ml of recHBsAg.

The secretion of IL-2 was measured by stimulation of the proliferation of VDA2 cells and concentration of IL-2 is is expressed as Stimulation Index (SI); the secretion of INF-g was measured using a commercial kit and expressed in pg/ml.

It was found that the secretion of IL-2 is dramatically increased by lower dose of 3D-MPL (7.5 µg(mcg)) and a maximal effect is obtained for 15 µg(mcg) of 3D-MPL.

The secretion of iL-2 is generally more important at 24h than at 48 or 72 h.

The secretion of INF-g is absent when HBsAg is adsorbed on Al(OH)3 in absence of 30-MPL. A small dose (7.5 µg(mcg) of 3D-MPL induces a secretion of INF-g and again, the maximal effect is obtained for 15 µg(mcg) of 30-MPL. Contrary to what is observed for IL-2, the secretion of INF-g is delayed in the culture and increases with time up to 96 hours.

Taken together these data indicate that 30-MPL (100 nm) is a potent inducer of Th1 when combined with HBsAg adsorbed on Al(OH)3.

### C. CONCLUSION

The effect of a formulation containing HBsAg adsorbed on Al(OH)3 and 3D-MPL on the induction of both humoral and cell-mediate immunity in Balb/c mice has been investigated. The results indicate that 3D-MPL clearly improves the kinetics of the anti-HBs response since much more anti-HBs antibodies are found after both the primary and secondary immunizations. The quality of the anti-HBs is also modified and a preferential induction of lgG2a has been observed, reflecting indirectly secretion of INF-g and thus induction of a cell-mediated immunity.

Direct evaluation of the induction of Th1 cells by formulations containing HBsAg, Al(OH)3 and 3D-MPL clearly indicates that 3D-MPL is a potent inducer of Th1 cells secreting both IL-2 and INF-g. This kind of formulation is thus important in the development of therapeutic vaccines.

For Tables showing the results of experiments described above, see Tables 1 to 6 below.

### Example 6

### CLINICAL USE OF 3D-MPL AS ADJUVANT TO HBsAg- -Preliminary results

### Study 3D-MPL-HBV-002 (ongoing)

This study compares HBsAg-3D-MPL to Engerix® -B in young healthy unprimed adult volunteers. End points
- Immunogenicity: magnitude of anti-HBs antibodies response
- Kinetics of anti-HBs antibody response
- Cell-mediated immunity induced by both vaccines (in vitro and in vivo).
- Reactogenicity, toxicology and safety.

### Material and methods

### a) Vaccines

| | **HBsAg (as in Engerix® B)** | **Alum** | **3D- MPL** |
|---|---|---|---|
| I | 20µg | 500µg | 50µg |
| II | 20µg | 500µg | None |

### b) Population

· Healthy adult volunteers, between 18 and 30 years of age.

· They must be negative for HBV markers and must never have been vaccinated against hepatitis B.

### c) Design

- Double-blind, randomized study.
- Schedule of vaccination : 0,1,6 months
- Follow-up till month 12.
- Blood sampling :
   * Anti-HBs antibody testing is done before, 7 days, 15 days. 30 days after each vaccination.
   * Biochemistry and haematology parameters are evaluated before ad 2 days after each dose.
   * Blood for evaluation of the cell-mediated immunity is taken before and after the primary vaccination course and the booster dose.
- Reactogenicity:

The subjects are required to record occurrence of local and general signs and symptoms on diary cards on the day of vaccination and during the 7 following days.

### Results

The results up to 2 months after the second dose are given below.

### Population :

A total of 29 subjects have given thee informed consent but only 27 - 15 male and 12 female - met the entry criteria. 15 subjects were allocated to Group I and 12 to Group II. The mean age was 22 years.

### Safety:

No serious adverse experience was reported and no subjects were withdrawn or had to be withdrawn from the study. No clinically significant modification of the haematological or biochemical parameters were observed. The incidence and severity of local and general signs and symptoms is similar in both vaccine groups.

### Immunogenicity:

The anti-HBs antibody titres have been measured up to day 90, ie 2 months after the second dose. The seroconversion rate (SC, in %) is defined as the appearance of antibodies in initially seronegative subjects.

The seroprotection rate is defined as the percentage of subjects with protective anti-HBs antibody titres. If there seems to be no clear difference in the magnitude of the antibody response with GMTs comparable between the two groups, the kinetics of the response is different and there is a dear advantage of the vaccine containing 3D-MPL. deed, 7 days after the second dose, 93% and 80% of the subjects receiving the 3D-MPL-HBV vaccine seroconverted and were protected respectively in comparison with a seroconversion rate of 95% and a seroprotection rate of 58% in the Engerix® -B group. This difference is maintained up to two months after the second dose and is particularly evident for the seroprotection rate. At this time point, all subjects who received the 3D-MPL-HBV vaccine are protected in comparison with 58% of the subjects vaccinated with the alum adjuvanted vaccine.

### Study 3D-MPL-HBV-003 (ongoing)

The material and methods are similar to those of 3D-MPL-HBV-002 study (see above). The schedules of vaccination are different:

| **Group** | **No of subjects** | **Vaccine** | **Schedule** |
|---|---|---|---|
| I | 25 | 30-MPL-HBsAg/alum | 0-2 months |
| II | 25 | Engerix® -B | 0-2 months |
| III | 25 | 3D-MPL-HBsAg/alum | 0-6 months |
| IV | 25 | Engerix® -B | 0-6 months |

### Results

Although the kinetics of the anti-HBs antibody response has not been followed as closely as in the previous study, it appears that the same conclusion can be drawn in the present study. The subjects who receive the 3D-MPL adjuvanted vaccine respond better after the second dose than those injected with the vaccine without 3D-MPL. All but one subject (95.8%) have protective titres one month after the second dose of 3D-MPL-HBV vaccine, as compared with 72.7% in the Engerix® -B group. Furthermore, in contrast with the previous trial, the 3D-MPL-HBV vaccine seems to induce higher antibody titres than Engerix® -B (214 and 72 mlU/ml respectively) indicating that a longer interval between the doses is perhaps important.

### Conclusions

In these two studies, the hepatitis B vaccine (containing HBsAg as in Engerix® -B and alum) adjuvanted with 3D-MPL was evaluated in healthy adult volunteers and compared to Engerix® -B (HBsAg S-antigen formulated with alum) according to various schedules. If there seems to be no difference in immunogenicity between the two vaccines after one dose, the 3D-MPL-HBV vaccine shows a clear advantage after the second dose, especially with a much higher percentage of subjects with protective antibody titres. The GMTs are also higher in this group when the two doses are given at two months interval instead of one month.

This could indicate a better priming of the response when 3D-MPL is added to the antigen. Such a vaccine would therefore be of great value in slow or low responders to hepatitis B vaccination, such as older people or immunocompromised subjects.

For Tables showing the results of the experiments described above, see Tables 7 to 9 below.

**TABLE 1**

| **EFFECT OF INCREASING DOSES OF 3D-MPL (> 500 NM) ON IMMUNOGENICITY OF RECHBSAG ADSORBED ON AL(OH)**_{**3**} | | | | |
|---|---|---|---|---|
| Amount of 3D-MPL (µg(mcg)/dose) | Anti-HBs response | | | |
| | Total lgG | | lgG2a | |
| | Day 14 | Day 21 | Day 14 | Day 21 |
| 0* | 69 | 743 | 3.2 | 11 |
| 3.13 | 122 | 541 | 3.8 | 20 |
| 6.25 | 296 | 882 | 6.4 | 24 |
| 12.5 | 371 | 1359 | 10 | 48 |
| 25 | 456 | 1493 | 18 | 138 |
| 50 | 403 | 1776 | 33 | 242 |

| | | | | |
|---|---|---|---|---|
| *HBsAg on Al | | | | |

**TABLE 2**

| **COMPARISON OF 3 CLINICAL LOTS CONTAINING OR NOT 3D-MPL AUSAB RESPONSE** | | | | |
|---|---|---|---|---|
| Lot | Dose of HBsAg on Al(OH)₃ (µg(mcg)) | Dose of 3D-MPL (µg(mcg)) | GMT Anti-HBs (mlU/ml) | |
| DSAH16 | 2.5 | 0 | 0.75 | 15.1 |
| DSAR501 | 2.5 | 6.25 | 12.4 | 96.7 |
| DSAR502 | 2.5 | 6.25 | 41.9 | 89.2 |

**TABLE 3**

| **COMPARISON OF 2 CLINICAL LOTS CONTAINING OR NOT 3D-MPL (>500NM) ANTI-HBS IGG AND IGG2A RESPONSE** | | | | | | |
|---|---|---|---|---|---|---|
| Lot | Dose of HBsAg on Al(OH)₃ (µg(mcg)) | Dose of 3D-MPL (µg(mcg)) | Anti-HBs response | | | |
| | | | IgG | | IgG2a | |
| | | | d15 | d21 | d15 | d21 |
| DSAH16 | 2.5 | 0 | 20 | 178 | <5 | 5 |
| DSAR502 | 2.5 | 6.25 | 113 | 641 | <5 | 28 |

**TABLE 4**

| **EFFECT OF 3D-MPL (<100 NM) DOSE ON IMMUNOGENICITY Of RECHBsAG ADSORBED ON AL(OH)**_{**3**} | | | | | | | |
|---|---|---|---|---|---|---|---|
| Dose of HBsAg on Al(OH)₃ (µg(mcg)) | Dose oMPLf (<100nm) (µg(mcg)) | Anti-HBs response | | | | | |
| | | Total lg | | IgG | | lgG2a | |
| | | d15 | d21 | d15 | d21 | d15 | d21 |
| 1 | 0 | 30 | 637 | 67 | 516 | 15 | 99 |
| 1 | 3.12 | 312 | 2302 | 335 | 3532 | 167 | 1752 |
| 1 | 6.25 | 538 | 2719 | 856 | 3932 | 261 | 2521 |
| 1 | 12.5 | 396 | 2104 | 485 | 3625 | 125 | 1393 |
| 1 | 25.0 | 38 | 446 | 141 | 638 | 28 | 233 |

**TABLE 5**

| **EFFECT OF 3D-MPL (>500 NM) ON THE INDUCTION OF HBSAG SPECIFIC TH1 CELLS IN BALB/C MICE** | | | | | | | | | | |
|---|---|---|---|---|---|---|---|---|---|---|
| Dose of HBsAg (µg(mcg) /mouse) | Formulation | In vitro secretion of | | | | | | | | |
| | | IL-2(St) | | | INF-γ (pg/ml) | | | IL-4 (pg/ml) | | |
| | | 24h | 48h | 72h | 24h | 48h | 72h | 24h | 48h | 72h |
| 20 | FCA | 1.3 | 2.0 | 8.0 | <125 | <125 | 385 | NT | NT | NT |
| - | FCA | 0.7 | 1.8 | 0.7 | <125 | <125 | <125 | NT | NT | NT |
| 20 | Al(OH)₃ | 1.0 | 1.4 | 1.2 | <125 | <125 | <125 | <40 | <40 | <40 |
| 20 | Al(OH)₃₊ MPL 30mcg) | 2 | 38 | 10 | <125 | 280 | 280 | <40 | <40 | <40 |
| After immunization as described in the text, lymph node cells were cultured with 5 µg(mcg) rec HBsAg/ml for the indicated periods of time and the secretion of IL-2, INFγ- and IL-4 were measured using respectively VDA₂ T-cell line and 2 commercial ELISA kits. | | | | | | | | | | |

**TABLE 6**

| **EFFECT OF DIFFERENT DOSES OF 3D-MPL (<100 NM) ON THE INDUCTION OF HBsAG SPECIFIC TH1 CELLS** | | | | | | | | |
|---|---|---|---|---|---|---|---|---|
| Dose of HBsAg (µg(mcg) /mouse) | Formulation | In vitro secretion of | | | | | | |
| | | IL-2(SI) | | | INF-γ (pg/ml) | | | |
| | | 24h | 48h | 72h | 24h | 48h | 72h | 96h |
| 20 | 0 | 2.6 | 28 | 21.8 | <67 | <67 | <67 | <67 |
| 20 | 7.5 | 207 | 173 | 58 | <67 | 207 | 522 | 698 |
| 20 | 15 | 270 | 71 | 36 | 275 | 878 | 1249 | 1582 |
| 20 | 30 | 41 | 59 | 36 | <67 | <67 | <67 | 207 |

**TABLE 7**

| **3D-MPLHBV-002** | | | | | | |
|---|---|---|---|---|---|---|
| **GROUP** | **VACCINE** | **TIMING** | **N** | **SEROCONVER SION RATE** | **GMT** | **SEROPROTECTION RATE (%)** |
| I | 3D-MPL-HBsAg (20µg) | Pre | 15 | 0.0 | 0 | 0.0 |
| | | PI(d7) | 15 | 0.0 | | 0.0 |
| | | PI(d15) | 14 | 42.9 | 4 | 7.1 |
| | | PI(d30) | 15 | 40.0 | 13 | 26.7 |
| | | PII(d37) | 15 | 93.3 | 41 | 80.0 |
| | | PII(d45) | 15 | 100.0 | 42 | 73.3 |
| | | PII (d60) | 15 | 100.0 | 25 | 80.0 |
| | | PII (d90) | 15 | 100.0 | 44 | 100.0 |
| II | Engerix® -B (20µg) | Pre | 12 | 0.0 | 0 | 0.0 |
| | | PI (d7) | 12 | 0.0 | | 0.0 |
| | | PI (d15) | 12 | 33.3 | 26 | 25.0 |
| | | PI (d30) | 12 | 50.0 | 9 | 25.0 |
| | | PII (d37) | 12 | 75.0 | 37 | 58.3 |
| | | PII (d45) | 12 | 83.3 | 20 | 41.7 |
| | | PII (d60) | 12 | 66.7 | 36 | 50.0 |
| | | PII (d90) | 12 | 83.3 | 31 | 58.3 |

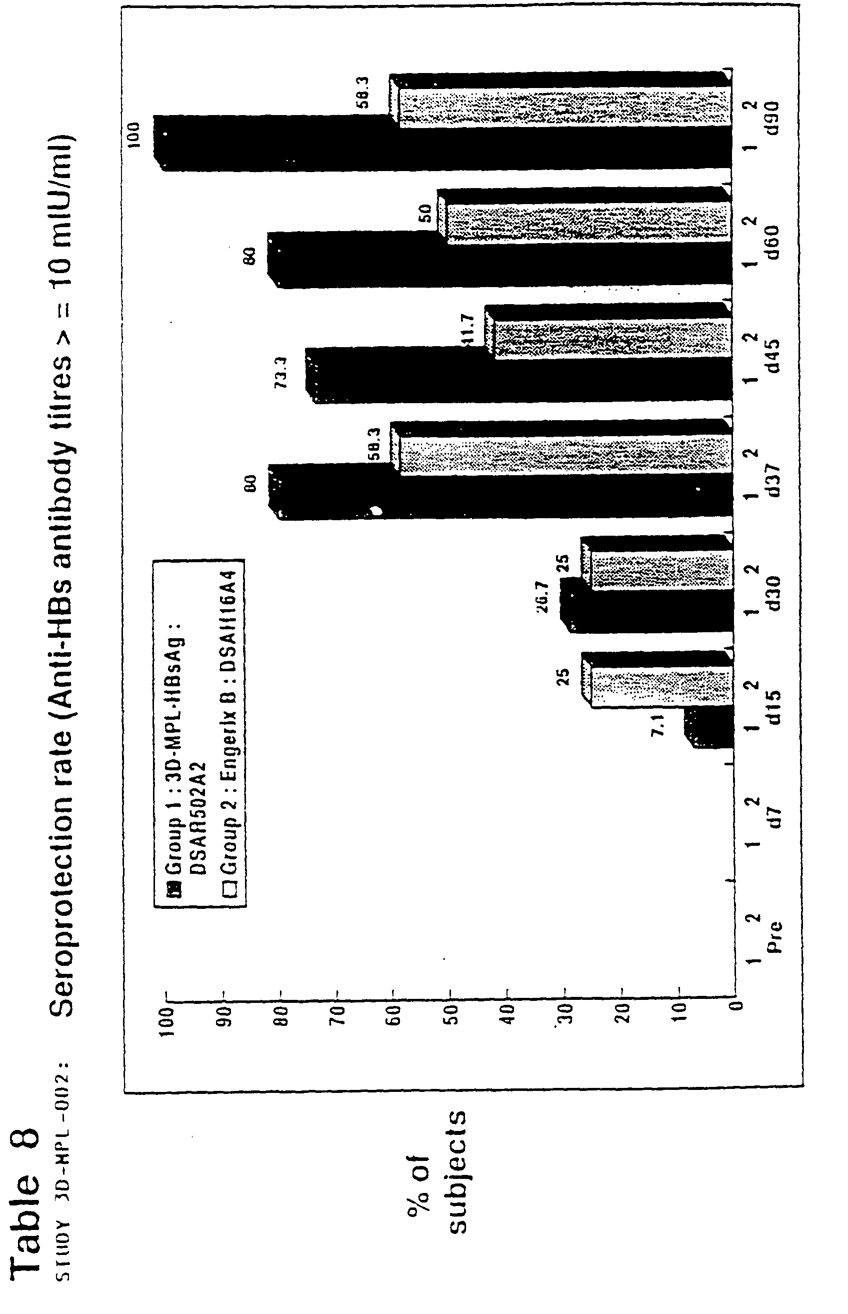

**TABLE 9**

| **3D-MPL-HBV-003 STUDY** | | | | | | |
|---|---|---|---|---|---|---|
| Group | Vaccine | Timing | N | SC(%) | GMT | SP (%) |
| I | 30-MPL-HBsAg | Pre | 25 | 0.0 | 0 | 0.0 |
| | (20µg) | P1(m1) | 23 | 56.5 | 10 | 26.1 |
| | | P1(m2) | 24 | 66.7 | 6 | 16.7 |
| | | P2(m3) | 24 | 100.0 | 214 | 95.8 |
| II | Engerix® -B (20µg) | Pre | 25 | 0.0 | 0 | 0.0 |
| | | P1(m1) | 24 | 41.7 | 12 | 29.2 |
| | | P1(m2) | 19 | 52.6 | 4 | 5.3 |
| | | P2(m3) | 22 | 90.9 | 72 | 72.7 |

## Claims

1. A vaccine composition comprising an antigen selected from:
a hepatitis B surface antigen wherein the hepatitis B surface antigen (HBsAg) is not part
of an immunogenic hybrid polypeptide;
in conjunction with 3-0-deacylated monophosphoryl lipid A and an aluminium salt.

2. A vaccine composition as claimed in Claim 1 in which the aluminium salt is aluminium hydroxide.

3. A vaccine composition as claimed in claim 1 or claim 2 which additionally comprises a hepatitis A antigen comprising live attenuated virus particles or inactivated attenuated virus particles

4. A vaccine composition as claimed in claim 3 wherein the Hepatitis A antigen is an inactivated whole cell composition derived from the HM-175 strain.

5. A vaccine composition as claimed in any preceding claim wherein the HBsAg comprises the 226 amino acid S antigen of HBsAg.

6. A vaccine composition as claimed in Claim 5 wherein the HBsAg additionally comprises a pre-S sequence.

7. A vaccine composition as claimed in Claim 6 in which the HBsAg comprises a composite particle of the formula (L*,S) wherein L* denotes a modified L protein of hepatitis B virus having an amino acid sequence comprising residues 12-52 followed by residues 133-145 followed by residues 175-400 of the L protein and S denotes the S-protein of HBsAg.

8. A vaccine composition as claimed in any preceding claim admixed with at least one other component selected from a non-hepatitis antigen which affords protection against one or more of the following: diphtheria, tetanus, pertussis, Haemophilus influenza b (Hib), and polio.

9. A vaccine composition according to Claim 8 selected from a DTP (diphtheriatetanus-pertussis) HBsAg combination, a Hib-HBsAg combination, a DTP-Hib-HBsAg combination and an IPV (inactivated polio vaccine) -DTP-Hib-HBsAg combination.

10. A vaccine composition as claimed in any preceding claim wherein the 3-O-deacylated monophosphoryl lipid A is present in the range 10µg-100µg per dose.

11. A vaccine composition as claimed in any preceding claim for use as a medicament.

12. Use of a composition as defined in any one of claims 1 to 10 in the manufacture of a medicament for the prophylaxis or treatment of hepatitis B infections.

## Patentansprüche

1. Impfstoffzusammensetzung, umfassend ein Antigen ausgewählt aus:
einem Hepatitis-B-Oberflächenantigen, wobei das Hepatitis-B-Oberflächenantigen (HBsAg) nicht Bestandteil eines immunogenen Hybridpolypeptids ist;
in Verbindung mit 3-O-desacyliertem Monophosphoryl-Lipid A und einem Aluminiumsalz.

2. Impfstoffzusammensetzung nach Anspruch 1, in der das Aluminiumsalz Aluminiumhydroxid ist.

3. Impfstoffzusammensetzung nach Anspruch 1 oder 2, die zusätzlich ein Hepatitis-A-Antigen enthält, umfassend lebende attenuierte Viruspartikel oder inaktivierte attenuierte Viruspartikel.

4. Impfstoffzusammensetzung nach Anspruch 3, in der das Hepatitis-A-Antigen eine Zusammensetzung aus inaktivierten ganzen Zellen ist, die sich von dem Stamm HM-175 ableitet.

5. Impfstoffzusammensetzung nach einem der vorangehenden Ansprüche, in der HBsAg das 226 Aminosäuren lange S-Antigen von HBsAg umfasst.

6. Impfstoffzusammensetzung nach Anspruch 5, in der das HBsAg zusätzlich eine Prä-S-Sequenz umfasst.

7. Impfstoffzusammensetzung nach Anspruch 6, in der das HBsAg ein zusammengesetztes Teilchen der Formel (L*, S) ist, wobei L* ein modifiziertes L-Protein des Hepatitis-B-Virus bedeutet, das eine Aminosäuresequenz mit den Resten 12-52, gefolgt von den Resten 133-145, gefolgt von den Resten 175-400 des L-Proteins umfasst, und S das S-Protein von HBsAg bedeutet.

8. Impfstoffzusammensetzung nach einem der vorangehenden Ansprüche im Gemisch mit mindestens einem weiteren Bestandteil, ausgewählt aus einem Non-Hepatitis-Antigenbestandteil, der gegen ein(e) oder mehrere der folgenden Krankheiten oder Krankheitserreger Schutz verleiht: Diphtherie, Tetanus, Pertussis, Hämophilus influenza b (Hib) und Polio.

9. Impfstoffzusammensetzung nach Anspruch 8, die eine DTP (Diphtherie-Tetanus-Pertussis)-HBsAg-, eine Hib-HBsAg-, eine DTP-Hib-HBsAg- oder eine IPV (inaktivierter Polioimpfstoff)-DTP-Hib-HBsAg-Kombination ist.

10. Impfstoffzusammensetzung nach einem der vorangehenden Ansprüche, in der das 3-O-desacylierte Monophosphoryl-Lipid A in einer Menge von 10 *µ*g bis 100*µ*g pro Dosis vorliegt.

11. Impfstoffzusammensetzung nach einem der vorangehenden Ansprüche zur Verwendung als Medikament.

12. Verwendung einer Zusammensetzung gemäß der Definition in einem der Ansprüche 1 bis 10 für die Herstellung eines Medikaments zur Prophylaxe oder Behandlung von Hepatitis-B-Infektionen.

## Revendications

1. Composition de vaccin comprenant un antigène choisi à partir de l'antigène de surface de l'hépatite B, dans lequel ledit antigène de surface de l'hépatite B (HBsAg) ne fait pas partie d'un polypeptide hybride immunogène ;
en conjonction avec le monophosphoryl-lipide A 3-O-désacylé, et un sel d'aluminium.

2. Composition de vaccin selon la revendication 1, dans laquelle le sel d'aluminium est l'hydroxyde d'aluminium.

3. Composition de vaccin selon la revendication 1 ou la revendication 2, qui comprend en plus, un antigène de l'hépatite A comprenant des particules de virus vivant atténué ou des particules de virus atténué inactivé.

4. Composition de vaccin selon la revendication 3, dans laquelle l'antigène de l'hépatite A est sous la forme d'une composition inactivée de cellules entières dérivant de la souche HM-175.

5. Composition de vaccin selon l'une quelconque des revendications précédentes, dans laquelle le HBsAg comprend l'antigène S de HBsAg, à 226 acides aminés.

6. Composition de vaccin selon la revendication 5, dans laquelle le HBsAg comprend en plus, une séquence pré-S.

7. Composition de vaccin selon la revendication 6, dans laquelle le HBsAg est un particule composite de formule (L*,S) dans laquelle L* représente une protéine L modifiée du virus de l'hépatite B, ayant une séquence d'acides aminés comprenant des résidus 12-52, suivis de résidus 135-145, suivis de résidus 175-400 de la protéine L, et S représente la protéine S de HBsAg.

8. Composition de vaccin selon l'une quelconque des revendications précédentes, mélangée avec au moins un autre composant choisi à partir d'un antigène non-hépatite qui assure une protection contre une ou plusieurs des affections suivantes: diphtérie, tétanos, coqueluche, Haemophilus influenzae b (Hib), et polio.

9. Composition de vaccin selon la revendication 8, choisie parmi une combinaison DTP (diphtérie-tétanos-coqueluche)-HBsAg, une combinaison Hib-HBsAg, une combinaison DTP-Hib-HBsAg, et une combinaison IPV (vaccin polio inactivé)-DTP-Hib-HBsAg.

10. Composition de vaccin selon l'une quelconque des revendications précédentes, dans laquelle le monophosphoryl-lipide A 3-O-désacylé est présent à raison de 10 µg à 100 µg par dose.

11. Composition de vaccin selon l'une quelconque des revendications précédentes, destinée à l'emploi en tant que médicament.

12. Utilisation d'une composition selon l'une quelconque des revendications 1 à 10, dans la fabrication d'un médicament destiné à la prophylaxie ou au traitement des infections par l'hépatite B.
